(19) Europäisches Patentamt
European Patent Office
Office européen des brevets

(11) **EP 3 015 468 A1**

(12) **EUROPEAN PATENT APPLICATION**
published in accordance with Art. 153(4) EPC


(43) Date of publication:
**04.05.2016 Bulletin 2016/18**

(21) Application number: **14817788.4**

(22) Date of filing: **25.06.2014**

(51) Int Cl.:
***C07D 471/06*** *(2006.01)*

(86) International application number:
**PCT/JP2014/066810**

(87) International publication number:
**WO 2014/208586 (31.12.2014 Gazette 2014/53)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.06.2013 JP 2013133673**



(71) Applicant: **Daiichi Sankyo Co., Ltd.**
**Chuo-ku**
**Tokyo 103-8426 (JP)**

(72) Inventors:
- **KAJINO, Hisaki**
**Hiratsuka-shi**
**Kanagawa 254-0014 (JP)**
- **MICHIDA, Makoto**
**Hiratsuka-shi**
**Kanagawa 254-0014 (JP)**
- **TAKAHASHI, Yasuo**
**Hiratsuka-shi**
**Kanagawa 254-0073 (JP)**
- **KUWAHARA, Yasuhisa**
**Hiratsuka-shi**
**Kanagawa 254-0014 (JP)**

(74) Representative: **Fairbairn, Angus Chisholm**
**Marks & Clerk LLP**
**90 Long Acre**
**London WC2E 9RA (GB)**


# (54) METHOD FOR PRODUCING TRICYCLIC HETEROCYCLIC COMPOUND


(57) It is an object of the present invention to provide a method for producing a high-purity tricyclic heterocyclic compound represented by formula (I), which is useful as a production intermediate for a pharmaceutical agent, wherein the method is an efficient production method with short steps and with a high yield, which does not use, as raw materials, expensive bromopyruvate and highly harmful copper chromite, and does not need silica gel column chromatography that generates, as by-products, large quantities of waste. The present invention relates to production of Compound (I) by the following method.


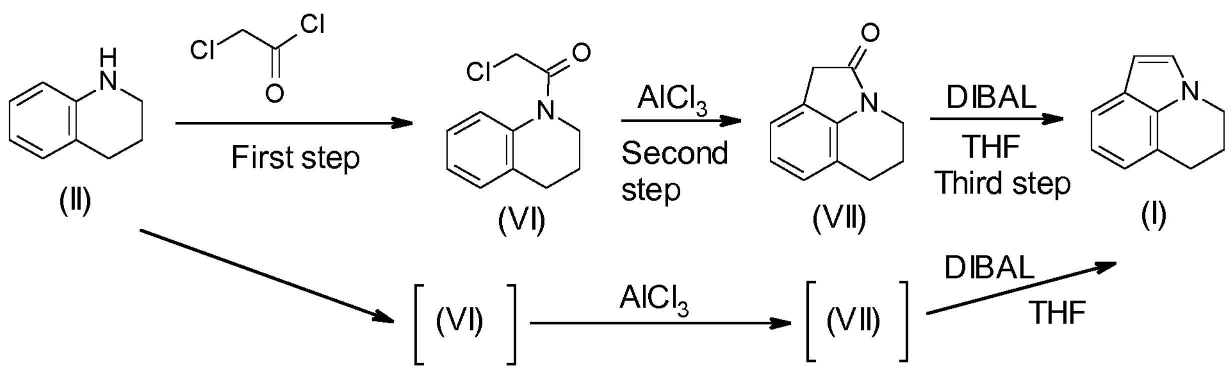


EP 3 015 468 A1

## Description

Technical Field

The present invention relates to a method for producing a tricyclic heterocyclic compound represented by the following formula (I):

[Formula 1]

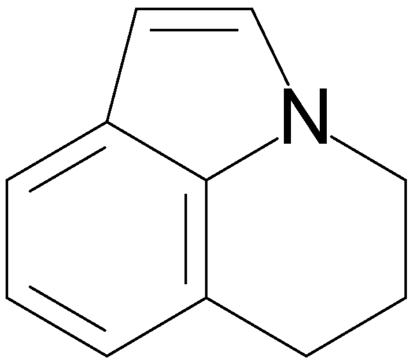

(I)

which is useful as a production intermediate for pharmaceutical agents.

Background Art

5,6-Dihydro-4H-pyrrolo[3,2,1-ij]quinoline (also known as: lilolidine) represented by the formula (I) is a compound that is highly useful as a synthetic intermediate for pharmaceutical products such as anticancer agents (see Patent Literatures 1 and 2). Moreover, Patent Literatures 1 to 3 disclose the following four steps for producing lilolidine.

[Formula 2]

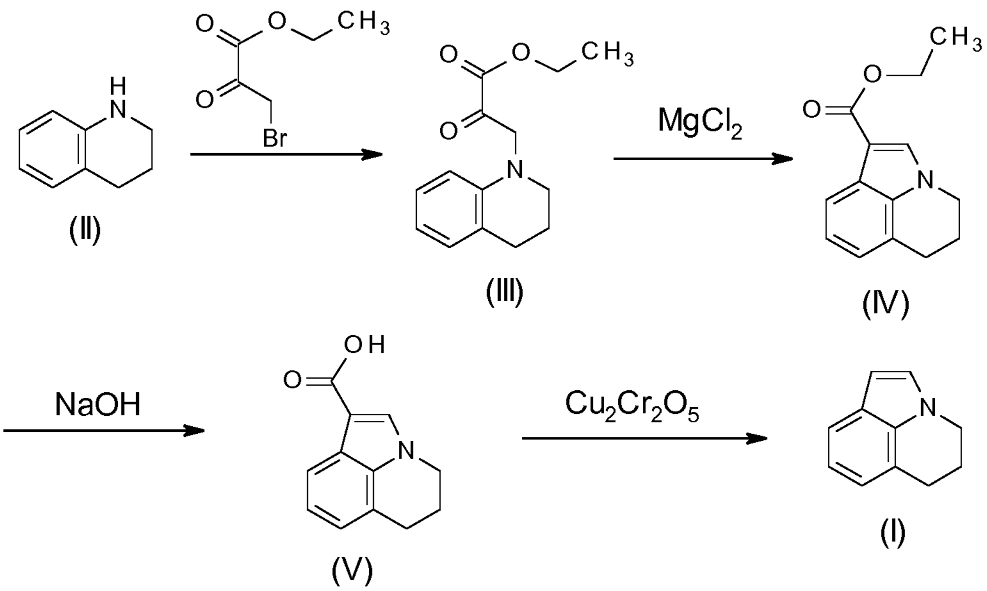


(II) (III) (IV) (V) (I)

The above steps are problematic in that expensive bromopyruvate and highly harmful copper chromite are used as raw materials, and in that the steps need silica gel column chromatography for purifying Compound (I) from a reaction mixture containing difficult to remove by-product, in which the silica gel column chromatography generates, as by-products, large quantities of waste. Accordingly, a method for producing high-purity Compound (I), more inexpensively,

by simple operations has been desired.

Patent Literature 4 describes a method for producing Compound (VI) by reacting 1,2,3,4-tetrahydroquinoline (Compound (II)) with chloroacetyl chloride. However, since a solvent and a base are used in this method, post-treatments such as neutralization, extraction and removal of the solvent are required after the reaction.

[Formula 3]

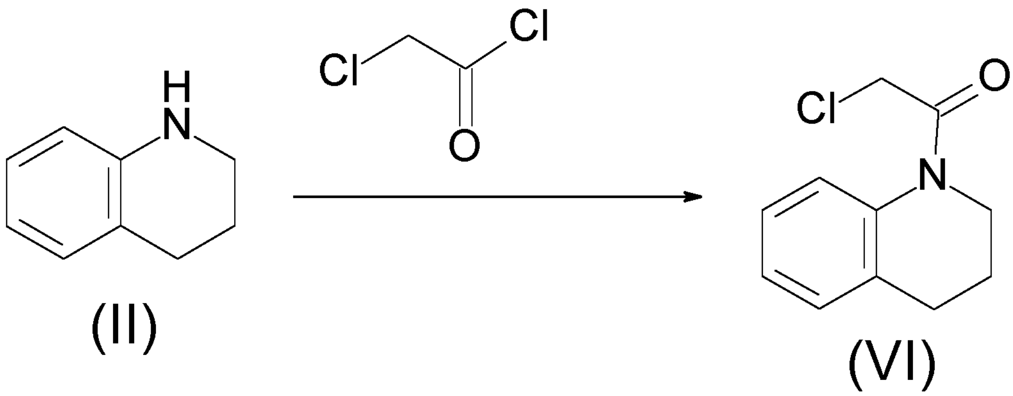

Patent Literature 5 and Non-patent Literature 1 describe a method for producing Compound (VII) by allowing aluminum chloride to act on Compound (VI), so as to produce Compound (VII) according to an intramolecular cyclization reaction.

[Formula 4]

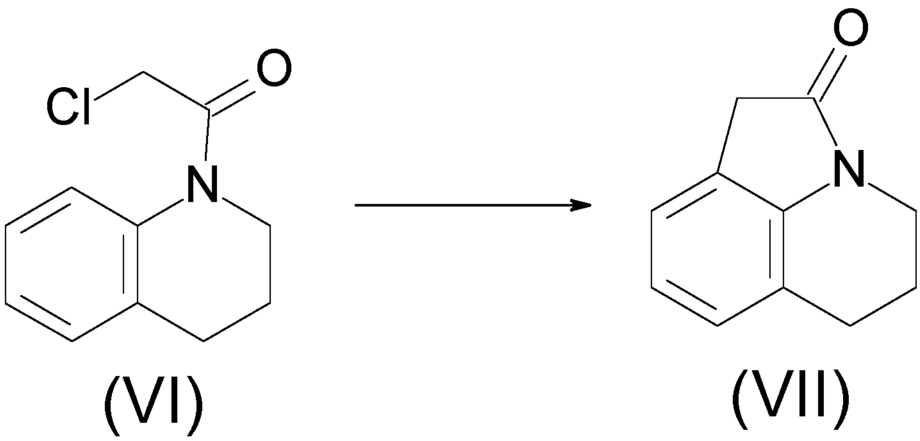

Non-patent Literature 2 describes a method for synthesizing an indole derivative by reduction of an N-alkyloxy indole derivative using lithium aluminum hydride or diisobutylaluminum hydride.

Citation List

Patent Literature

Patent Literature 1: International Publication No. WO 2003/076442
Patent Literature 2: International Publication No. WO 2006/086484
Patent Literature 3: Chinese Patent Laid-Open No. CN101302216
Patent Literature 4: U.S. Patent Application No. US2007/213321

Patent Literature 5: Japanese Patent Publication No. 52-048176

Non-patent Literature

Non-patent Literature 1: Yakugaku Zasshi (Journal of the Pharmaceutical Society of Japan), Vol. 64, No. 1, pp. 15 to 19

Non-patent Literature 2: Journal of Heterocyclic Chemistry, 25(3), pp. 937 to 942 (1998)

Summary of Invention

Technical Problem

In the conventional prior art, it has been difficult to obtain Compound (I) in a high yield and by simple operations. In particular, with regard to a purification method therefor, an industrially useful purification method for efficiently obtaining high-purity Compound (I) has so far not yet been found.

It is an object of the present invention to provide an industrially useful production method for producing high-purity Compound (I) by short steps and in a high yield, in which the production method does not use expensive bromopyruvate and highly harmful copper chromite, and does not need silica gel column chromatography for purifying Compound (I) from a reaction mixture containing difficult to remove by-products, in which the silica gel column chromatography generates, as by-product, large quantities of waste.

Solution to Problem

The present invention relates to:

(1) a method for producing Compound (I), which comprises reacting a compound represented by the following formula (II):

[Formula 5]

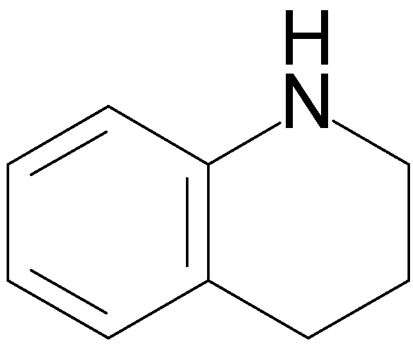

(II)

with chloroacetyl chloride (first step) to obtain a compound represented by the following formula (VI):

[Formula 6]

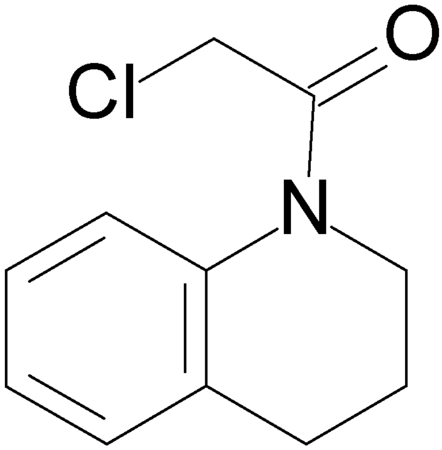

(VI)

then reacting Compound (VI) with aluminum chloride (second step) to obtain a compound represented by the following formula (VII):

[Formula 7]

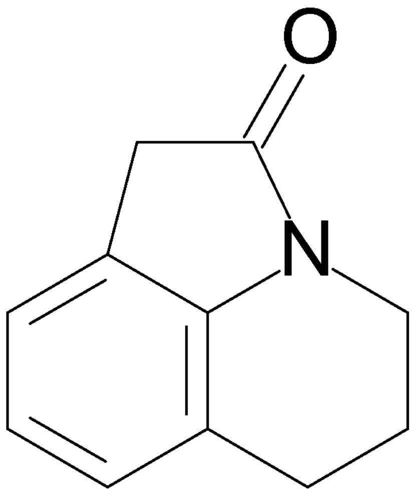

(VII)

and then reducing Compound (VII) with diisobutylaluminum hydride (hereinafter DIBAL) (third step); and
(2) the method according to (1), wherein the reaction in the first step is carried out in the absence of a base and in the absence of a solvent;
(3) the method according to (1) or (2), wherein the second step is carried out in the same reaction vessel as that in the first step;
(4) the method according to (3), wherein after completion of the first step, the second step is carried out without performing neutralization and extraction operations;
(5) the method according to any one of (1) to (4), wherein the reaction in the third step is carried out in the presence of tetrahydrofuran;
(6) the method according to any one of (1) to (5), wherein after completion of the reaction in the third step, Compound (I) is purified by steam distillation;
(7) the method according to (6), wherein the steam distillation is carried out in the presence of an organic solvent, while discharging an aqueous layer;
(8) the method according to (7), wherein the organic solvent is toluene;
(9) the method according to (6) or (7), wherein after the purification by steam distillation, highly polar impurities are removed by crystallization;
(10) a method for producing Compound (I), which comprises a step of purifying Compound (I) by steam distillation;
(11) the method according to (10), which comprises a step of purifying Compound (I) by performing steam distillation

in the presence of an organic solvent, while discharging an aqueous layer;
(12) the method according to (11), wherein the organic solvent is toluene; and
(13) the method according to any one of (10) to (12), wherein after the purification by steam distillation, highly polar impurities are removed by crystallization.

Advantageous Effects of Invention

According to the present invention, it becomes possible to obtain high-purity Compound (I) by short steps and in a high yield, without using, as raw materials, expensive bromopyruvate and highly harmful copper chromite, and without requiring silica gel column chromatography for purifying Compound (I) from a reaction mixture containing difficult to remove by-products, in which the silica gel column chromatography generates, as by-product, large quantities of waste.

Brief Description of Drawing

[Figure 1] Figure 1 shows a steam distillation in the present invention.

Description of Embodiments

1,2,3,4-Tetrahydroquinoline represented by formula (II), which is a starting material of the present invention, is a known compound.
A method of carrying out the present invention to produce Compound (I) is as follows.

[Formula 8]

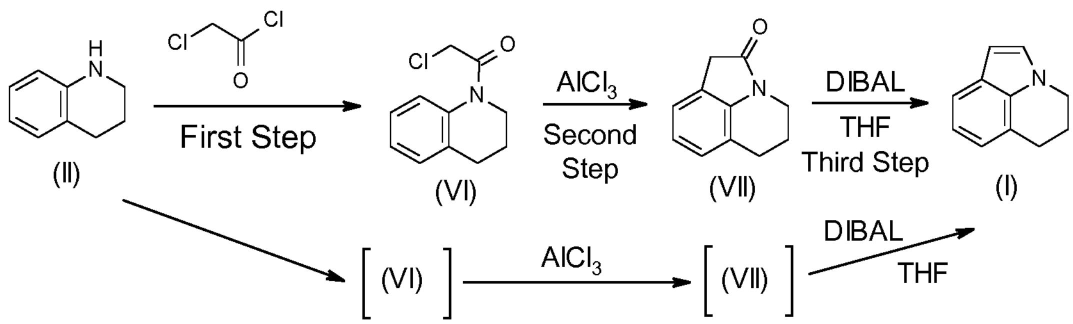


(First Step)

This step is a step of reacting Compound (II) with chloroacetyl chloride in the absence of a solvent and in the absence of a base, to produce Compound (VI). The amount of chloroacetyl chloride used in this step relative to Compound (II) is not particularly limited, as long as it is preferably 1 equivalent or more. It is more preferably 1 to 1.2 equivalents. The reaction temperature applied in this present step is preferably 60°C to 120°C, and more preferably 90°C to 110°C. The reaction time is preferably 0.5 hours to 5 hours, and more preferably 1 hour to 2 hours. Compound (VI) obtained in the present step can be used in the subsequent step without being isolated. Moreover, after completion of the reaction, the reaction solution is subjected to vacuum concentration or nitrogen ventilation, so that hydrogen chloride and excess chloroacetyl chloride can be removed. Furthermore, if Compound (VI) is used in the second step in a state in which chloroacetyl chloride remains, raw material remains, and the yield is reduced. Preferably, after completion of the reaction, the reaction solution is distilled under reduced pressure, so that hydrogen chloride and chloroacetyl chloride can be removed with good reproducibility.

(Second Step)

This step is a step of reacting Compound (VI) with aluminum chloride in the absence of a solvent to produce Compound (VII). The amount of aluminum chloride used in this step relative to Compound (VI) is preferably 1 to 3 equivalents, and more preferably 1.5 to 2.2 equivalents. The reaction temperature applied in the present step is preferably 80°C to 150°C, and more preferably 90°C to 110°C. The reaction time is preferably 0.5 hours to 5 hours, and more preferably 1 hour to 2 hours. Moreover, the reactions in the first step and the second step can be carried out in the same vessel, without performing post-treatments such as neutralization and extraction. In the case of Compound (VII) obtained

in this step, after completion of the reaction, the reaction solution is added to cold water to separate an organic layer, and the organic layer is washed with hydrochloric acid and then concentrated under reduced pressure, so that the obtained Compound (VII) can be used in the third step without being purified.

(Third Step)

this step is a step of reacting Compound (VII) with diisobutylaluminum hydride (DIBAL) in an inert solvent and in the presence of tetrahydrofuran (THF) to produce Compound (I). In this step, the reaction can be promoted by addition of THF. If this step is carried out in the absence of THF, raw material remains and the yield is reduced. The amount of DIBAL used in this step relative to Compound (VII) is generally 1 equivalent or more, and preferably 1.5 to 2.0 equivalents. The amount of THF used in this step relative to Compound (II) is not particularly limited, as long as it is 0.5 times or more than the amount of Compound (II). It is preferably used in an amount 1 time the amount of Compound (II). The inert solvent used in this step is not particularly limited, as long as Compound (VII) is dissolved therein to a certain extent, and the inert solvent does not prevent the reaction. Examples of such an inert solvent include: hydrocarbons such as toluene and hexane; and ethers such as THF. The inert solvent is preferably toluene or THF. The reaction temperature applied in this step is preferably -20°C to 10°C, and more preferably 0°C to 5°C. The reaction time is preferably 0.5 hours to 5 hours, and more preferably 1 hour to 2 hours.

(Purification Step)

Compound (I) of the present invention can be further purified by an ordinary method such as silica gel treatment, recrystallization, vacuum distillation, or steam distillation. The present Compound (I) can be preferably purified by steam distillation. More preferably, after steam distillation, a crystallization operation can be carried out in combination. By such a vacuum distillation or steam distillation operation, the generated product can be easily separated from reaction by-products. However, since Compound (I) tends to decompose under heating conditions, it is desirable to carry out the operation at a low temperature. However, lilolidine has a melting point of 80 to 85°C, and thus, if the operation is carried out at the melting point or below, it is likely that pipes in the distillation equipment will be clogged. As described above, when the distillation is carried out in a temperature range that satisfies stability and operability, special high-vacuum equipment is required for carrying out the vacuum distillation, and it results in a low recovery rate, and thus, it is difficult to carry out the operation. On the other hand, since steam distillation is carried out under ordinary pressure, it can be carried out in commonly used equipment at a relatively low temperature. Thus, stability can be ensured, and the recovery rate is also high.

Since the partial pressure ratio between steam and Compound (I) is approximately 120:1, it is necessary to separate Compound (I) from a mixture of Compound (I) and a large amount of water. Steam is continuously introduced into a solution of Compound (I) in an organic solvent that has been obtained by extraction performed after completion of the reaction, so that the inside of the vessel is filled with heated steam, and discharging heated steam is cooled in a condenser, so that the product of interest, together with the water and the organic solvent, can be captured by a receiver. The mixed solution is stirred in the receiver, Compound (I) is extracted into an organic layer, and the aqueous layer is discharged from the bottom of the receiver. By repeating this operation, a solution of Compound (I) in an organic solvent can be obtained. By adding common salt or saline into the receiver, extraction can be efficiently carried out. By such the steam distillation, low polar impurities can be effectively removed. The organic solvent used in the steam distillation of the present invention is not particularly limited, as long as it is not miscible with water. Examples of such an organic solvent include: aromatic hydrocarbons such as benzene, toluene, or xylene; aliphatic hydrocarbons such as pentane, hexane, or cyclohexane; halogenated hydrocarbons such as dichloromethane, chloroform, carbon tetrachloride, 1,2-dichloroethane, chlorobenzene, or dichlorobenzene; esters such as ethyl formate, ethyl acetate, propyl acetate, butyl acetate, or diethyl carbonate; or ethers such as diethyl ether, diisopropyl ether, tetrahydrofuran, 1,4-dioxane, dimethoxyethane, or tert-butyl methyl ether. Preferred examples of the organic solvent are aromatic hydrocarbons, and the organic solvent is more preferably toluene.

Subsequently, crystallization is carried out, so that highly polar impurities that are mixed in small amounts can be efficiently removed. With regard to the crystallization, specifically, highly polar impurities, which could not be removed by the steam distillation, are removed by performing crystallization in a 2-propanol/water system, as a result of solvent substitution of the organic solvent solution obtained by the steam distillation, so that high-purity Compound (I) can be obtained at a high recovery rate. Steam is directly blown into the crude product of Compound (I) concentrated to dryness to perform the steam distillation, and crystals precipitated from water are collected by filtration, so that Compound (I) can also be obtained without using solvents. In this case, however, there are cases where small amounts of highly polar impurities are mixed in the crystals.

Examples

Hereinafter, the present invention will be specifically described by way of Examples and Comparative Examples, but the present invention is not limited thereto.

[Example 1]

Production of 5,6-dihydro-4H-pyrrolo[3,2,1-ij]quinoline (Compound (I)) (purified by steam distillation and toluene extraction method)

(First Step)

Chloroacetyl chloride (50.88 g, 0.451 mol) was heated to approximately 70°C, 1,2,3,4-tetrahydroquinoline (50 g, 0.375 mol) was added dropwise thereto over 10 minutes, and the resulting mixture was then stirred at 100°C for 30 minutes. The reaction mixture was concentrated under reduced pressure, and hydrogen chloride and excess chloroacetyl chloride were distilled off, so as to obtain a concentrated solution of 2-chloro-1-(3,4-dihydro-2H-quinolin-1-yl)ethanone (Compound (VI)).

(Second Step)

To the obtained concentrated solution of Compound (VI), aluminum chloride (84.97 g, 0.637 mol) was added by divided addition at approximately 80 to 100°C, over approximately 2 hours, and the resulting mixture was further stirred for 1 hour. The reaction mixture was cooled to approximately 70°C, toluene (100 mL) was added thereto, and the resulting mixture was then cooled to 10°C and added dropwise to a mixed solution of toluene (250 mL), water (250 mL) and 6 N-hydrochloric acid (100 mL), which had previously been cooled to 10°C. After completion of the dropwise addition, the reaction vessel was fully washed with toluene (100 mL), the mixed solution was warmed to 35°C, and an organic layer was separated and then concentrated under reduced pressure to a volume of 250 mL, so as to obtain a solution of 5,6-dihydro-4H-pyrrolo[3,2,1-ij]quinolin-2(1H)-one (Compound (VII)) in toluene.

(Third Step)

To the obtained solution of Compound (VII) in toluene, tetrahydrofuran (50 mL) was added, the mixed solution was cooled to approximately 0°C, a 25% diisobutylaluminum hydride/toluene solution (321.14 g, 0.565 mol) was added dropwise to the reaction solution over approximately 1 hour 20 minutes, and the resulting mixture was further stirred for 3 hours. The reaction mixture was added dropwise over approximately 30 minutes into a mixed solution of 6 N-hydrochloric acid (125 mL) and water (125 mL), which had previously been cooled to approximately 0°C, the resulting mixture was then warmed to 40°C, and an organic layer was separated, washed with 1N-hydrochloric acid (150 mL) and 5% aqueous sodium bicarbonate (150 mL) in this order, and concentrated under reduced pressure to a volume of 300 mL, so as to obtain a solution of 5,6-dihydro-4H-pyrrolo[3,2,1-ij]quinoline (Compound (I)) in toluene.

(Purification Step)

Steam corresponding to a volume of approximately 5.5 L relative to water was blown into the obtained solution of Compound (I) in toluene over 7 hours, so as to carry out steam distillation. During the distillation, the concentration of sodium chloride in the aqueous layer of the receiver was adjusted to 0.1% or more by adding 20% saline, as needed. Thereafter, liquid separation was carried out, as appropriate, and the aqueous layer was discharged. The obtained toluene solution was concentrated under reduced pressure to a volume of 150 mL, 2-propanol (250 mL) and water (100 mL) were added to the solution, and the resulting mixture was concentrated under reduced pressure again to a volume of 150 mL. 2-Propanol (110 mL) was added to the reaction solution, and the resulting mixture was warmed to approximately 70°C for dissolution. After that, water (190 mL) was added to the resulting solution, and the resulting mixture was cooled to 0°C and stirred for 1 hour. The obtained suspension was filtered, and the residue was washed with a solution of 40% 2-propanol in water (80 mL) and dried under reduced pressure at 40°C for 4 hours to obtain 44.24 g of the title compound (Compound (I), purity: 100%, yield from 1,2,3,4-tetrahydroquinoline: 74.9%) in the form of white crystals.

[Example 2]

Production of 5,6-dihydro-4H-pyrrolo[3,2,1-ij]quinoline (Compound (I)) (purified by steam distillation and toluene extraction method)

(First Step)

Chloroacetyl chloride (25.4 kg, 224.9 mol) was heated to approximately 80°C, 1,2,3,4-tetrahydroquinoline (25.0 kg, 187.7 mol) was added dropwise thereto over approximately 1 hour, and the resulting mixture was then stirred at approximately 100°C for 30 minutes. The reaction mixture was concentrated under reduced pressure, and hydrogen chloride and excess chloroacetyl chloride were distilled off, so as to obtain a concentrated solution of 2-chloro-1-(3,4-dihydro-2H-quinolin-1-yl)ethanone (Compound (VI)).

(Second Step)

To the obtained concentrated solution of Compound (VI), aluminum chloride (42.5 kg, 318.7 mol) was added by divided addition at approximately 80 to 100°C, over approximately 3 hours, and the resulting mixture was further stirred for 1 hour. The reaction mixture was cooled to approximately 70°C, toluene (43.5 kg) was added thereto, and the resulting mixture was then cooled to approximately 0 to 10°C and added dropwise to a mixed solution of toluene (108.8 kg), water (175 L) and concentrated hydrochloric acid (30 kg), which had previously been cooled to approximately 0 to 10°C. After completion of the dropwise addition, the reaction vessel was fully washed with toluene (43.5 kg), the temperature was increased to approximately 35°C, and an organic layer was separated. Thereafter, the solution was concentrated under reduced pressure to a volume of approximately 125 L, so as to obtain a solution of 5,6-dihydro-4H-pyrrolo[3,2,1-ij]quinolin-2(1H)-one (Compound (VII)) in toluene.

(Third Step)

To the obtained solution of Compound (VII) in toluene, tetrahydrofuran (22.3 kg) was added, the mixed solution was cooled to -3°C to 0°C, a 25.4% diisobutylaluminum hydride/toluene solution (157.6 kg, 281.5 mol) was added dropwise to the reaction solution over approximately 4 hours, and the resulting mixture was further stirred for 1 hour. The reaction mixture was added dropwise over approximately 30 minutes into a mixed solution of hydrochloric acid (40.2 kg) and water (88 L), which had previously been cooled to 0 to 5°C. After that, the reaction vessel was fully washed with toluene (21.8 kg), the mixed solution was warmed to approximately 40°C, and an organic layer was separated, washed with 1N-hydrochloric acid (75 kg) twice and further with 5% aqueous sodium bicarbonate (75 kg) once, and concentrated under reduced pressure to a volume of approximately 175 L, so as to obtain a solution of 5,6-dihydro-4H-pyrrolo[3,2,1-ij]quinoline (Compound (I)) in toluene.

(Purification Step)

Steam corresponding to a volume of approximately 150 L relative to water was blown into the obtained solution of Compound (I) in toluene over 30 hours, so as to carry out steam distillation. During the distillation, the concentration of sodium chloride in the aqueous layer of the receiver was adjusted to 0.1% or more by adding 20% saline, as needed. Thereafter, liquid separation was carried out, as appropriate, and the aqueous layer was discharged. The obtained toluene solution was concentrated under reduced pressure to a volume of 75 L, 2-propanol (98.6 kg) and water (50 L) were added to the solution, and the resulting mixture was further concentrated under reduced pressure to a volume of 75 L. 2-Propanol (43.3 kg) was added to the concentrated solution, and the resulting mixture was warmed to approximately 70°C and stirred for 30 minutes. Water (95 L) was added to the reaction solution, and the resulting mixture was cooled to approximately 0 to 5°C and stirred for approximately 1 hour. Thereafter, the mixture was filtered, and crystals were washed with a solution of 40% 2-propanol in water (50 L) and dried under reduced pressure at approximately 40°C for approximately 33 hours to obtain 21.94 kg of the title compound (Compound (I), purity: 99.9%, yield from 1,2,3,4-tetrahydroquinoline: 74.3%) in the form of white crystals.

[Reference Example 1]

(1) Production of crude crystal of 5,6-dihydro-4H-pyrrolo[3,2,1-ij]quinoline (Compound (I)) (First, Second and Third Steps)

(First Step)

Under a nitrogen gas stream, chloroacetyl chloride (100 g, 0.813 mol) was heated to 90°C, 1,2,3,4-tetrahydroquinoline (113.7 g, 0.854 mol) was added dropwise thereto over approximately 2 hours, and the resulting mixture was then stirred at 110°C to obtain a reaction solution of 2-chloro-1-(3,4-dihydro-2H-quinolin-1-yl)ethanone (Compound (VI)).

(Second Step)

To the obtained reaction solution of Compound (VI), aluminum chloride (162.6 g, 1.220 mol) was added over approximately 2 hours, and the resulting mixture was further stirred for 1 hour. The reaction mixture was cooled to 70°C, toluene (500 mL) was added thereto, and the resulting mixture was then added dropwise to a mixed solution of toluene (1 L) and water (750 mL), which had previously been cooled to 0°C. An organic layer was separated, and an aqueous layer was then extracted again with toluene (500 mL). Organic layers were gathered and concentrated under reduced pressure to a volume of 1 L, so as to obtain a solution of 5,6-dihydro-4H-pyrrolo[3,2,1-ij]quinolin-2(1H)-one (Compound (VII)) in toluene.

(Third Step)

The obtained solution of Compound (VII) in toluene was cooled to -20°C, tetrahydrofuran (100 mL) was added to the reaction solution, a 25% diisobutylaluminum hydride/toluene solution (1.09 L, 1.626 mol) was added dropwise to the mixed solution over approximately 1 hour, and the resulting mixture was further stirred for 1 hour. The reaction mixture was added dropwise over 30 minutes into 6 N-hydrochloric acid (500 mL) which had previously been cooled to 0°C, and an organic layer was then separated and concentrated under reduced pressure to a volume of 500 mL. 2-Propanol (500 mL) was added to the concentrated solution, the resulting mixture was concentrated under reduced pressure to a volume of 300 mL, 2-propanol (500 mL) was further added to the reaction solution, and the resulting mixture was concentrated under reduced pressure to a volume of 500 mL. The concentrated solution was cooled to 0°C, water (500 mL) was then added dropwise thereto, and the resulting mixture was stirred at room temperature for 16 hours and then filtered. Crystals were washed with a solution of 50% 2-propanol in water (200 mL) and dried under reduced pressure at 30°C for 16 hours, so as to obtain 106.54 g of the title compound (Compound (I), purity: 92.0%, yield from 1,2,3,4-tetrahydroquinoline: 74.9%) in the form of light yellow crude crystals.

(2) Silica gel and activated carbon purification method for 5,6-dihydro-4H-pyrrolo[3,2,1-ij]quinoline (Compound (I)) (purification step)

The crude crystal (2 g) of Compound (I) obtained by the method of (1) was dissolved in heptane (20 mL), activated carbon (0.2 g) was added to the solution, and the resulting mixture was stirred at 50°C for 15 minutes. Silica gel (1 g) was further added to the reaction mixture, and the resulting mixture was stirred for 10 minutes. After that, solids were filtered off, and the filtrate was concentrated to a volume of 10 mL. 2-Propanol (20 mL) was added to the mixture, and the resulting mixture was concentrated again to a volume of 10 mL. This operation was carried out repeatedly twice, the reaction mixture was cooled to 0°C, and cold water (10 mL) was then added dropwise to the reaction solution. The crystallized crystals were filtered, washed with water (10 mL), and dried under reduced pressure at 30°C for 16 hours, so as to obtain 1.42 g of the title compound (Compound (I), purity: 99.1%, yield from 1,2,3,4-tetrahydroquinoline: 57.1%) in the form of white crystals.

[Reference Example 2]

Production of 5,6-dihydro-4H-pyrrolo[3,2,1-ij]quinoline (Compound (I)) (Third Step, purified by vacuum distillation)

(First Step)

Under a nitrogen gas stream, chloroacetyl chloride (104.2 g, 0.885 mol) was heated to approximately 70°C, 1,2,3,4-tetrahydroquinoline (117.9 g, 0.885 mol) was added dropwise thereto over approximately 30 minutes, and the resulting mixture was then stirred at approximately 80°C for 1 hour to obtain a reaction solution of 2-chloro-1-(3,4-dihydro-2H-quinolin-1-yl)ethanone (Compound (VI)).

(Second Step)

To the obtained reaction solution of Compound (VI), aluminum chloride (177.09 g, 1.328 mol) was added by divided addition at approximately 80 to 100°C over approximately 3 hours, and the resulting mixture was further stirred for 1 hour. The reaction mixture was cooled to approximately 40°C, toluene (500 mL) was added thereto, the resulting mixture was then cooled to approximately 10°C and added dropwise to water (500 mL) which had previously been cooled to approximately 10°C, and toluene (1000 mL) was further added thereto. An organic layer was separated, and the solution was then concentrated under reduced pressure to a volume of 1000 mL, so as to obtain a solution of 5,6-dihydro-4H-pyrrolo[3,2,1-ij]quinolin-2(1H)-one (Compound (VII)) in toluene.

(Third Step)

To the obtained solution of Compound (VII) in toluene, tetrahydrofuran (100 mL) was added. The solution was cooled to 0°C, a 25% diisobutylaluminum hydride/toluene solution (820 mL, 1.771 mol) was added dropwise thereto over approximately 1 hour 30 minutes, and the resulting mixture was further stirred for 30 minutes. The reaction mixture was added dropwise over approximately 40 minutes into a mixed solution of 6 N-hydrochloric acid (250 mL) and water (250 mL), which had previously been cooled to 0°C, the resulting mixture was then warmed to approximately 40°C, and an organic layer was separated and concentrated to a volume of 300 mL, so as to obtain a solution of 5,6-dihydro-4H-pyrrolo[3,2,1-ij]quinoline (Compound (I)) in toluene (reaction yield of Compound (I) from 1,2,3,4-tetrahydroquinoline: 70.2%).

(Purification Step)

The obtained solution of Compound (I) in toluene was subjected to vacuum distillation. The temperature of all pipes in the distillation device was kept at 85°C to 90°C, and distillation was carried out at an external temperature of 100 to 120°C at a degree of vacuum of 0.18 mmHg over 22 hours, so as to obtain 71.8 g of the title compound (Compound (I), purity: 95.3%, yield from 1,2,3,4-tetrahydroquinoline: 52.1%) in the form of white crystals.

[Example 3]

Production of 5,6-dihydro-4H-pyrrolo[3,2,1-ij]quinoline (Compound (I)) (purified by steam distillation)

(First Step)

Chloroacetyl chloride (101.76 g, 0.901 mol) was heated to approximately 70°C, 1,2,3,4-tetrahydroquinoline (100 g, 0.751 mol) was added dropwise thereto over approximately 30 minutes, and the resulting mixture was then stirred at approximately 100°C for 1 hour. The reaction mixture was concentrated under reduced pressure, and hydrogen chloride and excess chloroacetyl chloride were distilled off, so as to obtain a concentrated solution of 2-chloro-1-(3,4-dihydro-2H-quinolin-1-yl)ethanone (Compound (VI)).

(Second Step)

To the obtained concentrated solution of Compound (VI), aluminum chloride (170.19 g, 1.276 mol) was added by divided addition at approximately 80 to 100°C, over approximately 2 hours, and the resulting mixture was further stirred for 1 hour. The reaction mixture was cooled to approximately 60°C, toluene (200 mL) was added thereto, and the resulting mixture was then cooled to approximately 10°C and added dropwise to a mixed solution of toluene (500 mL), water (500 mL) and 6 N-hydrochloric acid (200 mL), which had previously been cooled to approximately 10°C. An organic layer was separated, and the solution was then concentrated under reduced pressure to a volume of 500 mL, so as to obtain a solution of 5,6-dihydro-4H-pyrrolo[3,2,1-ij]quinolin-2(1H)-one (Compound (VII)) in toluene.

(Third Step)

To the obtained solution of Compound (VII) in toluene, tetrahydrofuran (100 mL) was added. The solution was cooled to 0°C, a 25% diisobutylaluminum hydride/toluene solution (640.96 g, 1.127 mol) was added dropwise to the reaction solution over approximately 1 hour 30 minutes, and the resulting mixture was further stirred for 30 minutes. The reaction mixture was added dropwise over approximately 40 minutes into a mixed solution of 6 N-hydrochloric acid (250 mL) and water (250 mL), which had previously been cooled to 0°C, the resulting mixture was then warmed to approximately 40°C, and an organic layer was separated, washed with 1N-hydrochloric acid (300 mL) twice and further with 5% sodium

bicarbonate water (300 mL) once, and concentrated to dryness to obtain Compound (I).

(Purification Step)

Steam corresponding to a volume of approximately 11 L relative to water was blown into the obtained Compound (I) concentrated to dryness over 14 hours, so as to carry out steam distillation. Crystals precipitated in the receiver were filtered, as needed, and dried under reduced pressure at room temperature for approximately 16 hours, so as to obtain 87.25 g of the title compound (Compound (I), purity: 98.0%, yield from 1,2,3,4-tetrahydroquinoline: 73.9%) in the form of white crystals.

[Example 4]

Measurement of purity of Compound (I)

The purity of Compound (I) was obtained based on the area percentage of a peak according to HPLC.

Analysis conditions

Column: CAPCELL PAK C18 MGII, 250 mm x 4.6 mm, I.D., 5 μm (Shiseido Company, Limited)
Column temperature: 40°C
Sampler temperature: 25°C
Detection wavelength: UV 265 nm
Mobile phase A: Solution of 5 mM ammonium acetate in water
Mobile phase B: Acetonitrile
Flow rate: 1.0 mL/min
Amount injected: 5 μL
Time required for analysis: 40 minutes

Gradient conditions:

| Time (min) | Mobile phase A (%) | Mobile phase B (%) |
|---|---|---|
| 0.0 | 40 | 60 |
| 25.0 | 5 | 95 |
| 30.0 | 5 | 95 |
| 31.0 | 40 | 60 |
| 40.0 | 40 | 60 |

Measurement of reaction yield and purity

The measurement of the reaction yield and the purity was carried out according to the following operations and calculation formulae.

Reaction yield: Approximately 100 mg of toluene extract of Compound (I) was precisely weighed, and it was precisely diluted with acetonitrile to a volume of 25 mL and analyzed. Using the obtained area, the reaction yield was calculated according to the following calculation formula.

Calculation formula: [weight (standard preparation) × area (sample)] / area (standard preparation) × [weight (extract) / weight (sample)] / theoretical yield of Compound (I) × 100

Purity: Approximately 10 mg of a standard preparation of Compound (I) and approximately 10 mg of a sample were each precisely weighed, and they were each precisely diluted with acetonitrile to a volume of 25 mL and analyzed. Using the obtained areas, the purity was calculated using the following calculation formula.

Calculation formula: [weight (standard preparation) × area (sample)] / [weight (sample) × area (standard preparation)] × 100

The results obtained by measuring yields and purities in Examples 1 to 3 and Reference Examples 1 and 2 are shown in Table 1.

Table 1

| Example No. | Purity | Yield |
|---|---|---|
| Example 1 | 100.0% | 74.9% |
| Example 2 | 99.9% | 74.3% |
| Reference Example 1(1) | 92.0% | 79.4% |
| Reference Example 1(2) | 99.1% | 57.1% |
| Reference Example 2 | 95.3% | 52.1% |
| Example 3 | 98.0% | 73.9% |

In Examples 1 and 2, Compound (I) having a yield of 74% or more and a purity of 99.9% or more could be obtained by combining steam distillation with crystallization. As shown in Reference Example 1(1), when Compound (I) was obtained by performing crystallization only in a 2-propanol/water system, the yield thereof was high (79.4%), but the purity thereof was low (92.0%). In Reference Example 1(2), the crystals obtained in Reference Example 1(1) were purified by silica gel and activated carbon treatments. As a result, the purity thereof was improved to 99.1%, but the yield thereof was significantly reduced to 57.1%. In Reference Example 2, Compound (I) was purified by vacuum distillation. As a result, the purity of the obtained Compound (I) was 95.3%, and the yield thereof was low (52.1%). In Example 3, a toluene extract of Compound (I) was concentrated to dryness, and steam distillation was attempted to be performed in the absence of toluene. As a result, crystals of Compound (I) were precipitated from water on the receiver side, and were filtered, so that the Compound (I) could be directly isolated in the form of crystals. In this case, the yield of Compound (I) was as high (73.9%) as those of Examples 1 and 2, and the purity of the obtained Compound (I) was 98.0%. From these results, it was found that high-purity Compound (I) was obtained at the highest yield by combining steam distillation with crystallization under the conditions of Examples 1 and 2, without using silica gel.

[Reference Example 3]

Comparison between the case of distilling off the hydrogen chloride and chloroacetyl chloride remaining in the first step under reduced pressure and the case of not distilling them off under reduced pressure, in terms of influence on the yield of Compound (VII) (1) Production of 5,6-dihydro-4H-pyrrolo[3,2,1-ij]quinolin-2(1H)-one (Compound (VII)) in the case of not distilling off the hydrogen chloride and chloroacetyl chloride remaining in the first step under reduced pressure (First Step and Second Step)

(First Step)

Chloroacetyl chloride (5.09 g, 0.045 mol) was heated to approximately 70°C, 1,2,3,4-tetrahydroquinoline (Compound (I)I, 5.00 g, 0.038 mol) was added dropwise thereto over approximately 30 minutes, and then the resulting mixture was stirred at approximately 100°C for 1 hour to obtain a reaction solution of 2-chloro-1-(3,4-dihydro-2H-quinolin-1-yl)ethanone (Compound (VI)).

(Second Step)

To the obtained reaction solution of Compound (VI), aluminum chloride (12.51 g, 0.094 mol) was added by divided addition at approximately 80 to 100°C over approximately 2 hours. The resulting mixture was further stirred for

1 hour, and thereafter, the amount of 5,6-dihydro-4H-pyrrolo[3,2,1-ij]quinolin-2(1H)-one (Compound (VII)) generated was evaluated based on HPLC area percentage (Compound (VII): 72.14%, Compound (VI): 23.17%).

(2) Production of 5,6-dihydro-4H-pyrrolo[3,2,1-ij]quinolin-2(1H)-one (Compound (VII)) in the case of distilling off the hydrogen chloride and chloroacetyl chloride remaining in the first step under reduced pressure (First Step and Second Step)

(First Step)

Chloroacetyl chloride (5.09 g, 0.045 mol) was heated to approximately 70°C, 1,2,3,4-tetrahydroquinoline (5.00 g, 0.038 mol) was added dropwise thereto over 10 minutes, and the resulting mixture was then stirred at 100°C for 30 minutes. The reaction mixture was concentrated under reduced pressure, and excess chloroacetyl chloride was distilled off, so as to obtain a concentrated solution of 2-chloro-1-(3,4-dihydro-2H-quinolin-1-yl)ethanone (Compound (VI)).

(Second Step)

To the obtained concentrated solution of Compound (VI), aluminum chloride (11.01 g, 0.083 mol) was added by divided addition at approximately 80 to 100°C over approximately 2 hours. The resulting mixture was further stirred for 1 hour, and thereafter, the amount of 5,6-dihydro-4H-pyrrolo[3,2,1-ij]quinolin-2(1H)-one (Compound (VII)) generated was evaluated based on HPLC area percentage (Compound (VII): 96.17%, Compound (VI): 0.54%).

[Reference Example 4]

Comparison between the case of using THF in the third step and the case of not using THF in the third step, in terms of influence on the yield of Compound (I)

(1) Isolation of 5,6-dihydro-4H-pyrrolo[3,2,1-ij]quinolin-2(1H)-one (Compound (VII))

A solution of 5,6-dihydro-4H-pyrrolo[3,2,1-ij]quinolin-2(1H)-one (Compound (VII)) in toluene, which had been synthesized using 1,2,3,4-tetrahydroquinoline (Compound (II), 5 g, 0.038 mol) by the methods described in the first step and second step of Example 1, was concentrated to dryness. Hexane (25 mL) was added to the resultant, and the resulting mixture was stirred at room temperature. The crystallized crystals were filtered, washed with hexane (5 mL), and dried under reduced pressure at 30°C for 16 hours, so as to obtain 5.36 g of the title compound (82.5%, relative to 1,2,3,4-tetrahydroquinoline) in the form of white crystals.

(2) Synthesis of 5,6-dihydro-4H-pyrrolo[3,2,1-ij]quinoline (Compound (I)), in which THF is used in Third Step

The 5,6-dihydro-4H-pyrrolo[3,2,1-ij]quinolin-2(1H)-one (Compound (VII), 5 g, 28.87 mmol) obtained in (1) was dissolved in toluene (35 mL), tetrahydrofuran (5 mL) was added to the solution, the resulting mixture was cooled to -20°C, and a 25% diisobutylaluminum hydride/toluene solution (42.9 mL, 43.30 mmol) was then added dropwise to the reaction solution at -10°C or less over 30 minutes. The reaction mixture was added dropwise to 6 N-hydrochloric acid (25 mL), and the resulting mixture was stirred at 40°C for 30 minutes. An organic layer was separated, and the amount of Compound (I) generated was then quantified by HPLC (reaction yield: 88.9%).

(3) Synthesis of 5,6-dihydro-4H-pyrrolo[3,2,1-ij]quinoline (Compound (I)), in which THF is not used in Third Step

The 5,6-dihydro-4H-pyrrolo[3,2,1-ij]quinolin-2(1H)-one (Compound (VII), 354 mg, 2.04 mmol) obtained in (1) was dissolved in toluene (5 mL), the solution was cooled to -20°C, a 25% diisobutylaluminum hydride/toluene solution (4.05 mL, 4.09 mmol) was then added dropwise to the reaction solution at -10°C or less over 15 minutes, and the resulting mixture was stirred at room temperature for 16 hours. 6 N-hydrochloric acid (2 mL) was added dropwise to the reaction mixture, and the resulting mixture was stirred at 40°C for 30 minutes. An organic layer was separated, and the amount of Compound (I) generated was then quantified by HPLC (reaction yield: 77.0%).

Industrial Applicability

According to the present invention, it becomes possible to produce high-purity Compound (I) by a method of obtaining the Compound (I) by short steps and in a high yield, in which expensive bromopyruvate and highly harmful copper chromite are not used as raw materials, and in which silica gel column chromatography that generates, as by-products, large quantities of waste is not required.

## Claims

1. A method for producing a compound represented by the following formula (I):

[Formula 4]

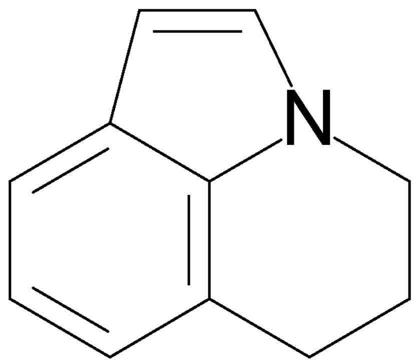

(I)

wherein the method comprises
reacting a compound represented by the following formula (II):

[Formula 1]

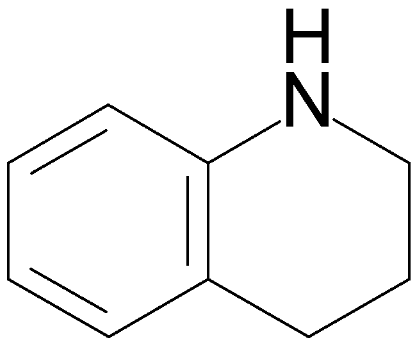

(II)

with chloroacetyl chloride (first step) to obtain a compound represented by the following formula (VI):

[Formula 2]

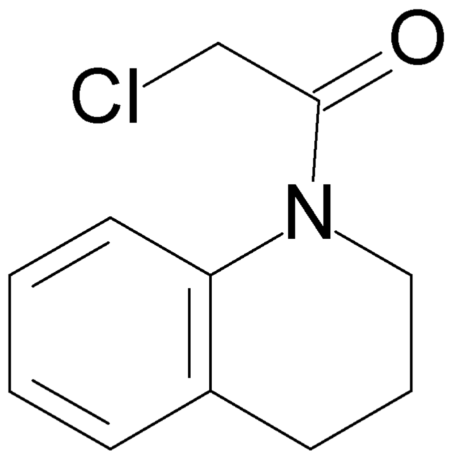

(VI)

then reacting Compound (VI) with aluminum chloride (second step) to obtain a compound represented by the following formula (VII):

[Formula 3]

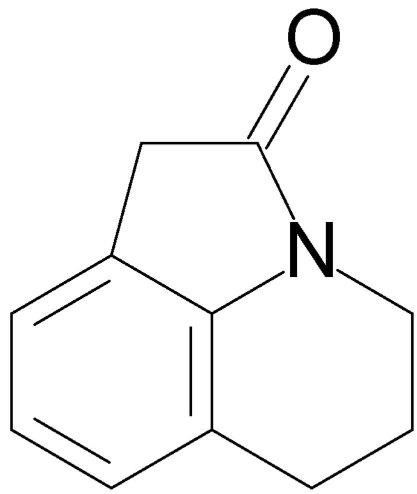

(VII)

and then reducing Compound (VII) with diisobutylaluminum hydride (third step).

**2.** The method according to claim 1, wherein the reaction in the first step is carried out in the absence of a base and in the absence of a solvent.

**3.** The method according to claim 1 or 2, wherein the second step is carried out in the same reaction vessel as that in the first step.

**4.** The method according to claim 3, wherein after completion of the first step, the second step is carried out without performing neutralization and extraction operations.

**5.** The method according to any one of claims 1 to 4,
wherein the reaction in the third step is carried out in the presence of tetrahydrofuran.

**6.** The method according to any one of claims 1 to 5,
wherein after completion of the reaction in the third step, Compound (I) is purified by steam distillation.

**7.** The method according to claim 6, wherein the steam distillation is carried out in the presence of an organic solvent, while discharging an aqueous layer.

8. The method according to claim 7, wherein the organic solvent is toluene.

9. The method according to claim 6 or 7, wherein after the purification by steam distillation, highly polar impurities are removed by crystallization.

10. A method for producing Compound (I), which comprises a step of purifying Compound (I) by steam distillation.

11. The method according to claim 10, which comprises a step of purifying Compound (I) by performing the steam distillation in the presence of an organic solvent, while discharging an aqueous layer.

12. The method according to claim 11, wherein the organic solvent is toluene.

13. The method according to any one of claims 10 to 12, wherein after the purification by steam distillation, highly polar impurities are removed by crystallization.

[Figure 1]

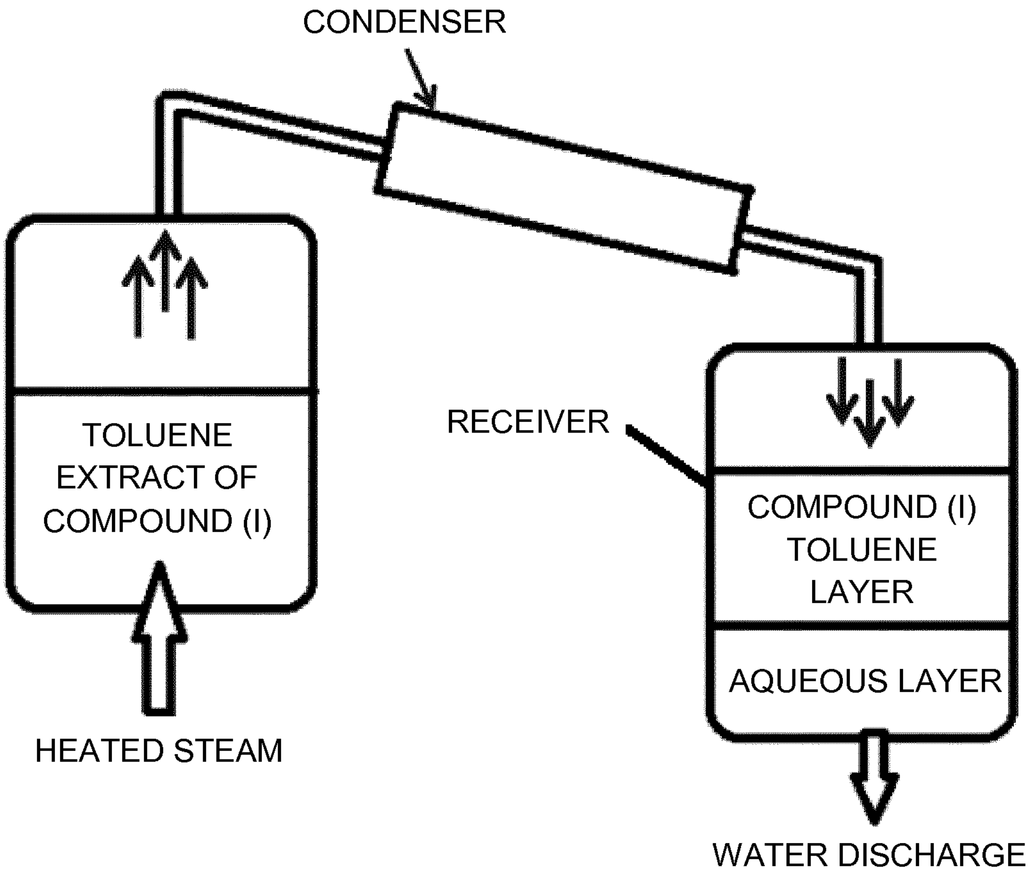
CONDENSER
TOLUENE
EXTRACT OF
COMPOUND (I)
RECEIVER
COMPOUND (I)
TOLUENE
LAYER
AQUEOUS LAYER
HEATED STEAM
WATER DISCHARGE

**INTERNATIONAL SEARCH REPORT**

International application No.
PCT/JP2014/066810

A. CLASSIFICATION OF SUBJECT MATTER
*C07D471/06*(2006.01)i

According to International Patent Classification (IPC) or to both national classification and IPC

B. FIELDS SEARCHED

Minimum documentation searched (classification system followed by classification symbols)
C07D471/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY/CASREACT(STN)

C. DOCUMENTS CONSIDERED TO BE RELEVANT

| Category* | Citation of document, with indication, where appropriate, of the relevant passages | Relevant to claim No. |
|---|---|---|
| Y/A | JP 2008-530026 A (Arqule, Inc.), 07 August 2008 (07.08.2008), entire text; particularly, item 2.2.1; example 1 & US 2006/0223760 A1 & EP 1846406 A1 & WO 2006/086484 A1 & KR 10-2007-0103047 A & CN 101133055 A | 1,3,4/2,5-13 |
| Y/A | CN 101302216 A (Shanghai Chempartner Co., Ltd.), 12 November 2008 (12.11.2008), entire text; particularly, claims; examples (Family: none) | 1,3,4/2,5-13 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>18 July, 2014 (18.07.14) | Date of mailing of the international search report<br>29 July, 2014 (29.07.14) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2014/066810

C (Continuation). DOCUMENTS CONSIDERED TO BE RELEVANT

| Category* | Citation of document, with indication, where appropriate, of the relevant passages | Relevant to claim No. |
|---|---|---|
| Y/A | JP 52-48176 B2 (Pfizer Corp.), 08 December 1977 (08.12.1977), entire text; particularly, general formulae II, IIB; column 9, line 37 to column 10, line 42; example 1 & US 3917838 A | 1,3,4/2 |
| Y/A | H. KATAYAMA et al., Preparation of hydrogenated azepino[3,2,1-hi]indoles, Journal of Heterocyclic Chemistry, 1988, Vol.25, No.3, pp.937-942 | 1,3,4/5 |
| A | JP 2010-521527 A (Neuraxon, Inc.), 24 June 2010 (24.06.2010), example 51 & US 2008/0234237 A1 & EP 2139886 A1 & WO 2008/116308 A1 & CN 101679397 A | 2 |
| A | JP 10-306095 A (Sankyo Co., Ltd.), 17 November 1998 (17.11.1998), paragraphs [0058], [0071] to [0077] & US 5773618 A & EP 807631 A1 & KR 10-1998-0079235 A & CN 1059904 C | 2 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003076442 A
- WO 2006086484 A
- CN 101302216
- US 2007213321 A
- JP 52048176 A


### Non-patent literature cited in the description


- **YAKUGAKU ZASSHI.** *Journal of the Pharmaceutical Society of Japan,* vol. 64 (1), 15-19
- *Journal of Heterocyclic Chemistry,* 1998, vol. 25 (3), 937-942